# EUROPÄISCHE PATENTSCHRIFT

(11) **EP 3 265 136 B1**
(45) Veröffentlichungstag und Bekanntmachung des Hinweises auf die Patenterteilung: **23.01.2019**
(21) Anmeldenummer: 16707634.8
(22) Anmeldetag: 29.02.2016
(51) Int. Cl.: A61L 2/18, A61L 2/22, B08B 3/02, B60B 19/00

(54) **VERFAHREN ZUR REINIGUNG EINES MULTIDIREKTIONALEN RADES**
METHOD FOR CLEANING A MULTIDIRECTIONAL WHEEL
PROCÉDÉ DE NETTOYAGE D'UNE ROUE MULTIDIRECTIONNELLE

(30) Priorität: 02.03.2015 DE 102015002531
(43) Veröffentlichungstag der Anmeldung: 10.01.2018
(73) Patentinhaber: Fresenius Medical Care Deutschland GmbH, 61352 Bad Homburg (DE)
(72) Erfinder: BRANDL, Matthias, 97631 Bad Königshofen (DE); FAULHABER, Thomas, 97493 Bergrheinfeld (DE); MELINIC, Pavel, 35390 Giessen (DE)
(74) Vertreter: Herrmann, Uwe
(86) Internationale Anmeldenummer: PCT/EP2016/000351
(87) Internationale Veröffentlichungsnummer: WO 2016/138985

(56) Entgegenhaltungen:
- DE-A1- 1 939 932
- US-A- 5 188 293
- US-A- 5 853 127
- US-A1- 2007 096 541
- US-A1- 2010 156 168

## Beschreibung

Die vorliegende Erfindung betrifft ein Verfahren zur Reinigung eines multidirektionalen Rades, wobei das Rad einen um eine Achse drehbaren Radkörper sowie eine Mehrzahl von Rotationskörpern aufweist, die sich am Umfang des Radkörpers befinden und die die Lauffläche des Rades bilden.

Aus dem Stand der Technik ist es bekannt, z. B. medizinische Geräte, wie beispielsweise Dialysegeräte mit Fahrrollen zu versehen, um deren Beweglichkeit zu gewährleisten. Als Rollen werden dabei beispielsweise Schwenk- bzw. Bockrollen eingesetzt. Bei dieser Art der Konstruktion sind die Rollen im Allgemeinen fest mit dem Maschinengestell verbunden. Soll eine solche Rolle nach einer Verunreinigung, z. B. durch Verschmutzungen auf dem Boden etc., gereinigt werden, ist dies mitunter nur schwer möglich.

Die Qualität einer solchen Reinigung der Rolle hängt wesentlich von der Gründlichkeit der Person ab, die die Reinigungstätigkeit vornimmt.

Sind die Rollen durch Abdeckungen etc. weitgehend oder komplett abgedeckt, wird die Reinigung der Rollen entsprechend schwieriger.

Aus dem Stand der Technik sind des Weiteren sogenannte multidirektionale Räder bekannt, d. h. Räder, mit denen eine Bewegung in mehr als eine Richtung möglich ist.

Ein solches multidirektionales Rad ist beispielsweise aus der WO 2011/113562 A2 bekannt.

Ein solches bekanntes multidirektionales Rad umfasst einen Radkörper sowie eine Mehrzahl von Rotationskörpern, insbesondere Walzen, die sich am Außenumfang des Radkörpers befinden. Dabei kann vorgesehen sein, dass sich die Längsachse der Walzen entweder in der Rotationsebene des Radkörpers oder in einem Winkel dazu erstreckt.

Des Weiteren wird Bezug genommen auf die DE 193 99 32 A1, AU 198 547 668, WO 00/12327 A1 und US 7,641,288, die ebenfalls derartige Radkonstruktionen bzw. multidirektionale Räder offenbaren.

Der vorliegenden Erfindung liegt die Aufgabe zugrunde, ein Verfahren bereitzustellen, mittels dessen ein multidirektionales Rad auf vergleichsweise einfache Art und Weise und gründlich gereinigt werden kann.

Diese Aufgabe wird durch ein Verfahren mit den Merkmalen des Anspruchs 1 gelöst. Danach ist vorgesehen, dass der Radkörper wenigstens einen Hohlraum aufweist, der wenigstens eine Einlassöffnung und wenigstens eine Auslassöffnung für eine Spülflüssigkeit aufweist. Das Verfahren umfasst den Schritt des Einleitens der Spülflüssigkeit, wie beispielsweise eines Desinfektionsmittels etc. durch die Einlassöffnung in den wenigstens einen Hohlraum des Radkörpers und das Umspülen der Rotationskörper durch die Spülflüssigkeit nach dem Austritt der Spülflüssigkeit aus der wenigstens einen Auslassöffnung.

Bei dem Rotationskörper kann es sich beispielsweise um eine Kugel oder Walze handeln. Handelt es sich um eine Walze, kann vorgesehen sein, dass deren Längsachse in der Rotationsebene des Radkörpers oder auch in einem Winkel dazu verläuft.

Erfindungsgemäß ist somit vorgesehen, dass in den Radkörper eine Spülflüssigkeit eingeleitet wird und diese Spülflüssigkeit dann durch den genannten Hohlraum des Radkörpers hindurch strömt und die Rotationskörper umströmt, sodass diese gereinigt bzw. desinfiziert werden können.

Dabei ist vorzugsweise vorgesehen, dass der Radkörper eine Mehrzahl von Hohlräumen aufweist und dass das Verfahren den Schritt des vorzugsweise gleichzeitigen Einleitens der Spulflüssigkeit in die mehreren Hohlräume umfasst. Auf diese Weise ist es möglich, an mehreren Stellen und vorzugsweise gleichzeitig Spülflüssigkeit in den Hohlraum bzw. in die Hohlräume des Radkörpers einzuleiten.

Denkbar ist es beispielsweise, dass die Spülflüssigkeit den Hohlraum des Radkörpers von innen nach außen, d.h. in radialer Richtung oder auch schräg dazu durchströmt.

In einer weiteren Ausgestaltung der Erfindung ist vorgesehen, dass das Verfahren das Aufsetzen oder Einsetzen eines Spüladapters auf oder in den Radkörper umfasst, wobei die Spülflüssigkeit aus dem Spüladapter in den wenigstens einen Hohlraum des Radkörpers strömt. So ist es denkbar, dass ein Spüladapter beispielsweise auf die wenigstens eine Einlassöffnung des Radkörpers aufgesetzt wird und die Spülflüssigkeit dann den Hohlraum des Radkörpers durchströmt und nach dem Austritt durch die Auslassöffnung(en) die Rotationskörper umströmt.

Dieser Spüladapter weist eine einzige Öffnung auf, aus der die Spülflüssigkeit austritt oder auch vorzugsweise eine Mehrzahl von Auslässen, durch die die Spülflüssigkeit austritt.

Vorzugsweise wird das Verfahren so durchgeführt, dass die Spülflüssigkeit den Hohlraum des Radkörpers von innen nach außen durchströmt. So ist es beispielsweise denkbar, dass der Radkörper eine zentrale Einlassöffnung aufweist und von dort aus die Spülflüssigkeit radial oder zur radialen Richtung schräg nach außen hin, d. h. in Richtung der Rotationskörper strömt.

Alternativ oder zusätzlich dazu kann vorgesehen sein, dass der Hohlraum derart ausgebildet ist, dass eine Durchströmung in Umfangsrichtung oder schräg dazu erfolgt. So ist es denkbar, dass die Spülflüssigkeit den Hohlraum des Radkörpers wenigstens abschnittsweise in Umfangsrichtung des Radkörpers durchströmt.

Eine Kombination der beiden vorgenannten Strömungsrichtungen (Richtungskomponenten in tangentialer und radialer Richtung) ist ebenfalls denkbar.

In einer weiteren Ausgestaltung der Erfindung ist vorgesehen, dass das Verfahren den Schritt des Abnehmens des Rades von einem Gerät, insbesondere von einem Dialysegerät, umfasst und dass die Spülflüssigkeit in das von dem Gerät abgenommenen Rad eingeleitet wird. So ist es beispielsweise denkbar, dass die Fahrrolle, d. h. das Rad z. B. mit einem Schnellverschluss von einem Nutzer von dem Gerät abgenommen werden kann. Je nach Gewicht des Gerätes kann dann z. B. eine klappbare Stütze die Stützfunktion der Rolle übernehmen. Bei leichteren Geräten bzw. Gestellen kann das gesamte Gestell auch auf die Seite gelegt werden.

Nach dem Abnehmen des Rades kann dann das Durchspülen des Rades vorgenommen werden, vorzugsweise unter Verwendung des o. g. Spüladapters.

In einer bevorzugten Ausgestaltung der Erfindung ist vorgesehen, dass die Spülflüssigkeit an mehreren unterschiedlichen Positionen in den Hohlraum bzw. in die Hohlräume des Radkörpers eingeleitet wird. Auf diese Weise ist eine besonders gründliche Reinigung der Hohlräume sowie auch der Rotationskörper möglich.

Diese mehreren Positionen können in Umfangsrichtung und/oder in radialer Richtung des Radkörpers voneinander beabstandet angeordnet sein.

Die vorliegende Erfindung betrifft des Weiteren ein multidirektionales Rad, wobei das Rad einen um eine Achse drehbaren Radkörper sowie eine Mehrzahl von Rotationskörpern aufweist, die sich am Umfang des Radkörpers befinden und die die Lauffläche des Rades bilden.

Erfindungsgemäß ist vorgesehen, dass der Radkörper wenigstens einen Hohlraum aufweist, der wenigstens eine Einlassöffnung und wenigstens eine Auslassöffnung für eine Spülflüssigkeit aufweist und dass die wenigstens eine Auslassöffnung für die Spülflüssigkeit im Bereich der Rotationskörper angeordnet ist.

In einer weiteren Ausgestaltung der Erfindung ist vorgesehen, dass in dem Radkörper eine Mehrzahl von Hohlräumen, insbesondere eine Mehrzahl von Kanälen vorgesehen ist, die im Bereich der Rotationskörper wenigstens eine Austrittsöffnung für die Spülflüssigkeit aufweisen.

Weiterhin kann vorgesehen sein, dass pro Rotationskörper genau eine Austrittsöffnung vorgesehen ist oder dass pro Rotationskörper mehrere Austrittsöffnungen vorgesehen sind.

Eine besonders gründliche Reinigung, insbesondere von walzenförmigen Rotationskörpern, lässt sich dann erreichen, wenn die Austrittsöffnung schlitzartig ausgeführt ist und sich vorzugsweise entlang des Rotationskörpers bzw. entlang von dessen Längsachse erstreckt.

Auch ist es denkbar, dass eine Mehrzahl von Austrittsöffnung vorgesehen ist, die sich vorzugsweise entlang der Längsachse des Rotationskörpers erstrecken. Auf diese Weise ist es möglich, die Spülflüssigkeit nicht nur an einer Stelle, sondern verteilt an mehreren Positionen auf die Oberfläche des Rotationskörpers, wie beispielsweise einer Walze oder dergleichen aufzubringen.

Die vorliegende Erfindung betrifft des Weiteren eine Vorrichtung umfassend wenigstens eine Quelle für eine Spülflüssigkeit, wenigstens einen mit der Quelle in Verbindung stehenden Spüladapter, der wenigstens eine Einlassöffnung, die mit der Quelle in Verbindung steht, und wenigstens eine Auslassöffnung zum Einleiten der Spülflüssigkeit in das Rad aufweist. Des Weiteren weist die Vorrichtung wenigstens ein Rad gemäß der vorliegenden Erfindung auf.

Vorzugsweise ist vorgesehen, dass der Spüladapter wenigstens einen und vorzugsweise eine Mehrzahl von rohrförmigen Abschnitten aufweist, die mit der Quelle für eine Spülflüssigkeit in Verbindung stehen. Diese rohrförmigen Abschnitte können in entsprechende Ausnehmungen des Radkörpers eingeführt werden und sodann kann der Spülvorgang eingeleitet werden.

In einer weiteren Ausgestaltung der Erfindung ist vorgesehen, dass die Auslassöffnungen des Spüladapters in Umfangsrichtung und/oder in radialer Richtung des Spüladapters voneinander beabstandet sind.

So ist es möglich, an mehreren verschiedenen Positionen des Radkörpers die Spülflüssigkeit einzuleiten.

Weitere Einzelheiten und Vorteile der Erfindung werden anhand eines in der Zeichnung beschriebenen Ausführungsbeispiels näher erläutert.

Es zeigen:
- Figur 1:: eine perspektivische Ansicht des unteren Bereichs eines Dialysegerätes mit einem abgenommenen multidirektionalen Rad gemäß der Erfindung und
- Figur 2:: eine perspektivische Ansicht des Spüladapters.

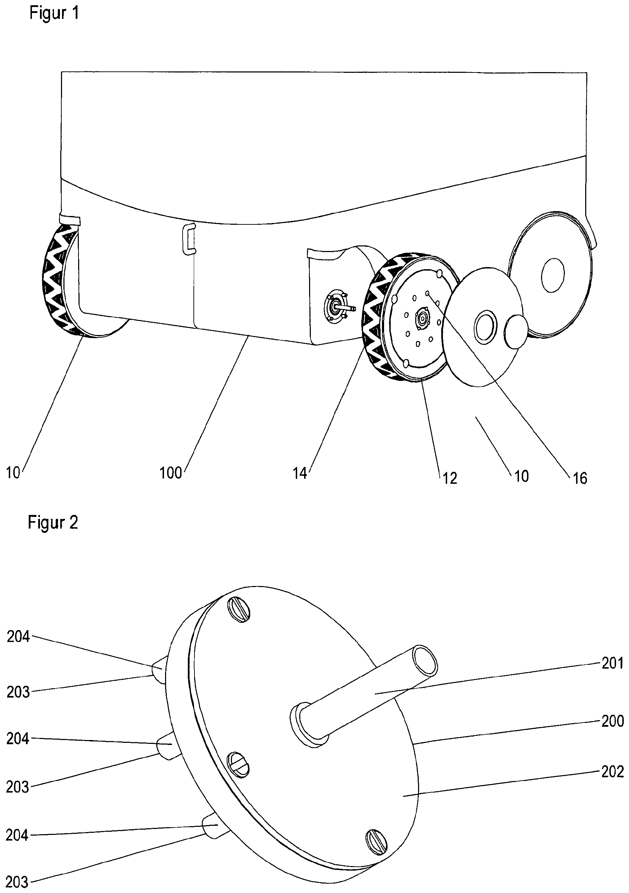

In Figur 1 ist mit dem Bezugszeichen 100 das Gehäuse eines medizinischen Gerätes, wie beispielsweise eines Dialysegerätes, gekennzeichnet.

Das Gehäuse 100 ist mit vier Rollen versehen, wobei zumindest zwei Rollen durch multidirektionale Räder gemäß der vorliegenden Erfindung gebildet werden.

Wie dies aus Figur 1 hervorgeht, bestehen diese Räder 10 aus einem Radkörper 12 und einer Mehrzahl von Rotationskörpern 14, die in Form von Walzen ausgebildet sind und sich entlang der Außenoberfläche des Radkörpers 12 erstrecken.

Wie dies aus Figur 1 hervorgeht, verlaufen die Längsachsen bzw. die Rotationsachsen der Walzen 14 nicht in der Rotationsebene, d. h. nicht in der Längsschnittebene des Radkörpers 12, sondern im Winkel dazu.

Aufgrund des Vorhandenseins der Rotationskörper 14 kann das Rad 10 nicht nur in der Richtung der Rotationsebene, d. h. der Längsschnittebene des Radkörpers 12, bewegt werden, sondern auch in einem Winkel dazu, entsprechend der Anordnung der Rotationskörper 14.

Wie dies weiter aus Figur 1 hervorgeht, befindet sich in dem Radkörper in Umfangsrichtung versetzt zueinander eine Mehrzahl von Einlassöffnungen für eine Spülflüssigkeit . Diese Einlassöffnungen sind mit dem Bezugszeichen 16 gekennzeichnet und kreisförmig angeordnet.

Abgesehen von dieser Anordnung ist es denkbar, diese Einlassöffnungen in anderer Art und Weise anzuordnen oder auch mehr oder weniger als die dargestellten acht Einlassöffnungen 16 pro Radkörper 12 zu verwenden.

Figur 2 zeigt in einer perspektivischen Ansicht von schräg oben einen Spüladapter 200. Dieser Spüladapter 200 weist einen Grundkörper 202 auf, der in dem hier dargestellten Ausführungsbeispiel die Form einer Kreisscheibe aufweist. Des Weiteren weist der Spüladapter 200 eine Vielzahl von Auslassöffnungen 203 auf, die sich in Endbereichen von stutzenförmigen Rohrabschnitten 204 erstrecken.

Das Bezugszeichen 201 kennzeichnet eine zentrale Einlassöffnung, die beispielsweise mit dem dargestellten Schlauch in Verbindung steht und durch die eine Spülflüssigkeit in den Innenbereich des Spüladapters 200 eintritt.

Nach dem Eintritt in den Innenbereich bzw. Hohlraum des Spüladapters 200 verteilt sich die Spülflüssigkeit auf die Rohrleitungen 204 und tritt durch die Auslassöffnungen 203 aus dem Spüladapter aus.

Nachdem der Nutzer das Rad 10 beispielsweise durch Lösen eines Schnellverschlusses von der in Figur 1 dargestellten Achse des Gerätes abgenommen hat, wird das Rad 10 mit dem Spüladapter 200 verbunden, indem die rohrförmigen Stutzen 204 in die Öffnungen 16 des Rades 10 eingeführt werden.

Anschließend wird die Spülflüssigkeit aus einer nicht dargestellten Quelle in den Spüladapter 200 und von diesem in den Innenbereich des Rades bzw. des Radkörpers 12 eingeleitet.

Von dort erstrecken sich Kanäle oder sonstige Hohlräume durch den Innenbereich des Radkörpers 12 nach außen, d. h. zu der Umfangsfläche hin, an der sich die Rotationskörper 14 befinden. Die Spülflüssigkeit tritt somit im Bereich der Umfangsfläche des Radkörpers 12 aus, an der auch die Rotationskörper 14 angeordnet sind.

Denkbar ist es, dass die Spülflüssigkeit in Umfangsrichtung verteilt an mehreren Positionen des Radkörpers 12 austritt. Bevorzugt ist es, dass für jeden Rotationskörper 14 wenigstens eine Austrittsöffnung vorgesehen ist, sodass gewährleistet ist, dass jeder Rotationskörper 14 mit einer Spülflüssigkeit und vorzugsweise mit einer Desinfektionsflüssigkeit beaufschlagt wird.

Vorzugsweise sind die Hohlräume bzw. Kanäle so ausgeführt, dass die Flüssigkeit von innen nach außen den kompletten Radkörper durchströmt und sämtliche Einzelrollen bzw. Rotationskörper komplett von Schmutz und Keimen befreit.

Auf diese Weise ist es möglich, trotz des vergleichsweise komplexen Aufbaus des dargestellten multidirektionellen Rades eine Reinigung bzw. Desinfektion des Radkörpers selbst sowie auch sämtlicher Rotationskörper zu erreichen, die sich auf der Außenfläche des Radkörpers befinden.

## Patentansprüche

1. Verfahren zur Reinigung eines multidirektionalen Rades (10), wobei das Rad (10) einen um eine Achse drehbaren Radkörper (12) sowie eine Mehrzahl von Rotationskörpern (14) aufweist, die sich am Außenumfang des Radkörpers (12) befinden und die die Lauffläche des Rades (10) bilden, **dadurch gekennzeichnet, dass** der Radkörper (12) wenigstens einen Hohlraum aufweist, der wenigstens eine Einlassöffnung (16) und wenigstens eine Auslassöffnung für eine Spülflüssigkeit aufweist, und dass das Verfahren das Einleiten einer Spülflüssigkeit durch die Einlassöffnung (16) in den wenigstens einen Hohlraum des Radkörpers (12) und das Umspülen der Rotationskörper (14) durch die Spülflüssigkeit nach dem Austritt der Spülflüssigkeit aus der Auslassöffnung umfasst.

2. Verfahren nach Anspruch 1, **dadurch gekennzeichnet, dass** der Radkörper (12) eine Mehrzahl von Hohlräumen aufweist und dass das Verfahren den Schritt des vorzugsweise gleichzeitigen Einleitens der Spülflüssigkeit in die mehreren Hohlräume umfasst.

3. Verfahren nach Anspruch 1 oder 2, **dadurch gekennzeichnet, dass** das Verfahren das Aufsetzen oder Einsetzen eines Spüladapters (200) auf oder in den Radkörper (12) umfasst, wobei die Spülflüssigkeit aus dem Spüladapter (200) in den wenigstens einen Hohlraum des Radkörpers (12) strömt.

4. Verfahren nach einem der vorhergehenden Ansprüche, **dadurch gekennzeichnet, dass** die Spülflüssigkeit den Hohlraum des Radkörpers (12) von innen nach außen durchströmt.

5. Verfahren nach einem der vorhergehenden Ansprüche, **dadurch gekennzeichnet, dass** die Spülflüssigkeit den Hohlraum des Radkörpers (12) in Umfangsrichtung des Radkörpers (12) durchströmt.

6. Verfahren nach einem der vorhergehenden Ansprüche, **dadurch gekennzeichnet, dass** das Verfahren den Schritt des Abnehmens des Rades (10) von einem Gerät (100), insbesondere von einem Dialysegerät, umfasst und dass die Spülflüssigkeit in das von dem Gerät (100) abgenommene Rad (10) eingeleitet wird.

7. Verfahren nach einem der vorhergehenden Ansprüche, **dadurch gekennzeichnet, dass** die Spülflüssigkeit an mehreren unterschiedlichen Positionen in den oder die Hohlräume des Radkörpers (12) eingeleitet wird.

8. Verfahren nach Anspruch 7, **dadurch gekennzeichnet, dass** die mehreren Positionen in Umfangsrichtung und/oder in radialer Richtung des Radkörpers (12) voneinander beabstandet sind.

9. Multidirektionales Rad (10), wobei das Rad (10) einen um eine Achse drehbaren Radkörper (12) sowie eine Mehrzahl von Rotationskörpern (14) aufweist, die sich am Außenumfang des Radkörpers (12) befinden und die die Lauffläche des Rades (10) bilden, **dadurch gekennzeichnet, dass** der Radkörper (12) wenigstens einen Hohlraum aufweist, der wenigstens eine Einlassöffnung und wenigstens eine Auslassöffnung für eine Spülflüssigkeit aufweist, dass die wenigstens eine Auslassöffnung für die Spülflüssigkeit im Bereich der Rotationskörper (14) angeordnet ist und, dass das Rad ausgebildet ist, das Einleiten einer Spülflüssigkeit durch die Einlassöffnung in den wenigstens einen Hohlraum des Radkörpers und das Umspülen der Rotationskörper durch die Spülflüssigkeit nach dem Austritt der Spülflüssigkeit aus der Auslassöffnung, zu ermöglichen.

10. Multidirektionales Rad nach Anspruch 9, **dadurch gekennzeichnet, dass** eine Mehrzahl von Hohlräumen, insbesondere eine Mehrzahl von Kanälen vorgesehen ist, die im Bereich der Rotationskörper (14) die wenigstens eine Auslassöffnung für die Spülflüssigkeit aufweisen.

11. Multidirektionales Rad nach Anspruch 9 oder 10, **dadurch gekennzeichnet, dass** pro Rotationskörper (14) genau eine Auslassöffnung vorgesehen ist oder dass pro Rotationskörper (14) mehrere Auslassöffnungen vorgesehen sind.

12. Multidirektionales Rad nach einem der Ansprüche 9 bis 11, **dadurch gekennzeichnet, dass** die Auslassöffnung schlitzartig ausgeführt ist und sich vorzugsweise entlang des Rotationskörpers (14) erstreckt, oder dass eine Mehrzahl von Auslassöffnungen vorgesehen ist, die vorzugsweise entlang des Rotationskörpers (14) verteilt sind.

13. Vorrichtung umfassend wenigstens eine Quelle für eine Spülflüssigkeit, wenigstens einen mit der Quelle in Verbindung stehenden Spüladapter (200), der wenigstens eine Einlassöffnung (201), die mit der Quelle in Verbindung steht, und wenigstens eine Auslassöffnung (203) zum Einleiten der Spülflüssigkeit in das Rad (10) aufweist, und wenigstens ein multidirektionales Rad (10) gemäß einem der Ansprüche 9 bis 12.

14. Vorrichtung nach Anspruch 13, **dadurch gekennzeichnet, dass** der Spüladapter (200) wenigstens einen, vorzugsweise eine Mehrzahl von rohrförmigen Abschnitten (204) aufweist, die mit der Quelle für eine Spülflüssigkeit in Verbindung stehen.

15. Vorrichtung nach Anspruch 13 oder 14, **dadurch gekennzeichnet, dass** die Auslassöffnungen (203) in Umfangsrichtung und/oder in radialer Richtung des Spüladapters (200) voneinander beabstandet sind.

## Claims

1. A method of cleaning a multidirectional wheel (10), wherein the wheel (10) has a wheel body (12) which is rotatable about an axis and has a plurality of rotating bodies (14) which are located at the outer periphery of the wheel body (12) and which form the running surface of the wheel (10), **characterized in that** the wheel body (12) has at least one hollow space which has at least one inlet opening (16) and at least one outlet opening for a flushing fluid; and **in that** the method comprises the introduction of a flushing fluid through the inlet opening (16) into the at least one hollow space of the wheel body (12) and the washing around of the rotating body (14) by the flushing fluid after the discharge of the flushing fluid from the outlet opening.

2. A method in accordance with claim 1, **characterized in that** the wheel body (12) has a plurality of hollow spaces; and **in that** the method comprises the step of the preferably simultaneous introduction of the flushing fluid into the plurality of hollow spaces.

3. A method in accordance with claim 1 or claim 2, **characterized in that** the method comprises the placing or inserting of a flushing adapter (200) on or into the wheel body (12), with the flushing fluid flowing out of the flushing adapter (200) into the at least one hollow space of the wheel body (12).

4. A method in accordance with one of the preceding claims, **characterized in that** the flushing fluid flows through the hollow space of the wheel body (12) from the inside to the outside.

5. A method in accordance with one of the preceding claims, **characterized in that** the flushing fluid flows through the hollow space of the wheel body (12) in the peripheral direction of the wheel body (12).

6. A method in accordance with one of the preceding claims, **characterized in that** the method comprises the step of removing the wheel (10) from a device (100), in particular from a dialysis device; and **in that** the flushing fluid is introduced into the wheel (10) removed from the device (100).

7. A method in accordance with one of the preceding claims, **characterized in that** the flushing fluid is introduced into the hollow space or spaces of the wheel body (12) at a plurality of different positions.

8. A method in accordance with claim 7, **characterized in that** the plurality of positions are spaced apart from one another in the peripheral direction and/or in the radial direction of the wheel body (12).

9. A multidirectional wheel (10), wherein the wheel (10) has a wheel body (12) which is rotatable about an axis and has a plurality of rotating bodies (14) which are located at the outer periphery of the wheel body (12) and which form the running surface of the wheel (10), **characterized in that** the wheel body (12) has at least one hollow space which has at least one inlet opening and at least one outlet opening for a flushing fluid; **in that** the at least one outlet opening for the flushing fluid is arranged in the region of the rotating bodies (14); and **in that** the wheel is configured to enable the introduction of a flushing fluid through the inlet opening into the at least one hollow space of the wheel body and the washing around of the rotating body by the flushing fluid after the discharge of the flushing fluid from the outlet opening.

10. A multidirectional wheel in accordance with claim 9, **characterized in that** a plurality of hollow spaces, in particular a plurality of channels, are provided which have the at least one outlet opening for the flushing fluid in the region of the rotating bodies (14).

11. A multidirectional wheel in accordance with claim 9 or claim 10, **characterized in that** exactly one outlet opening is provided per rotating body (14); or **in that** a plurality of outlet openings are provided per rotating body (14).

12. A multidirectional wheel in accordance with one of the claims 9 to 11, **characterized in that** the outlet opening is in the form of a slit and preferably extends along the rotating body (14); or **in that** a plurality of outlet openings are provided which are preferably distributed along the rotating body (14).

13. An apparatus comprising at least one source for a flushing fluid, at least one flushing adapter (200) which communicates with the source and which has at least one inlet opening (201), which communicates with the source, and at least one outlet opening (203) for introducing the flushing fluid into the wheel (10), and comprising at least one multidirectional wheel (10) in accordance with one of the claims 9 to 12.

14. An apparatus in accordance with claim 13, **characterized in that** the flushing adapter (200) has at least one tubular section, preferably a plurality of tubular sections (204), which communicate with the source for a flushing fluid.

15. An apparatus in accordance with claim 13 or claim 14, **characterized in that** the outlet openings (203) are spaced apart from one another in the peripheral direction and/or in the radial direction of the flushing adapter (200).

## Revendications

1. Procédé de nettoyage d'une roue (10) multidirectionnelle, la roue (10) comportant un corps de roue (12) rotatif sur un axe ainsi qu'une pluralité de corps de rotation (14), qui se trouvent sur la circonférence extérieure du corps de roue (12) et qui forment la surface de roulement de la roue (10), **caractérisé en ce que** le corps de roue (12) comporte au moins une cavité, qui comporte au moins un orifice d'entrée (16) et au moins un orifice de sortie pour un liquide de rinçage (16) et **en ce que** le procédé comprend l'introduction d'un liquide de rinçage par l'orifice d'entrée (16) dans l'au moins une cavité du corps de roue (12) et le baignage des corps de rotation (14) par le liquide de rinçage après la sortie du liquide de rinçage par l'orifice de sortie.

2. Procédé selon la revendication 1, **caractérisé en ce que** le corps de roue (12) comporte une pluralité de cavités et **en ce que** le procédé comprend l'étape de l'introduction de préférence simultanée du liquide de rinçage dans les plusieurs cavité.

3. Procédé selon la revendication 1 ou 2, **caractérisé en ce qu'**il comprend la pose ou l'insertion d'un adaptateur de rinçage (200) sur ou dans le corps de roue (12), le liquide de rinçage s'écoulant depuis l'adaptateur de rinçage (200) dans l'au moins une cavité du corps de roue (12).

4. Procédé selon l'une des revendications précédentes, **caractérisé en ce que** le liquide de rinçage traverse la cavité du corps de roue (12) de l'intérieur vers l'extérieur.

5. Procédé selon l'une des revendications précédentes, **caractérisé en ce que** le liquide de rinçage traverse la cavité du corps de roue (12) dans le sens circonférentiel du corps de roue (12).

6. Procédé selon l'une des revendications précédentes, **caractérisé en ce qu'**il comprend l'étape du retrait de la roue (10) d'un appareil (100), en particulier d'un appareil de dialyse, et **en ce que** le liquide de rinçage est introduit dans la roue (10) retirée de l'appareil (100).

7. Procédé selon l'une des revendications précédentes, **caractérisé en ce que** le liquide de rinçage est introduit dans la ou les cavités du corps de roue (12) dans plusieurs positions différentes.

8. Procédé selon la revendication 7, **caractérisé en ce que** les plusieurs positions sont espacées les unes des autres dans le sens circonférentiel et/ou dans le sens radial du corps de roue (12).

9. Roue (10) multidirectionnelle, la roue (10) comportant un corps de roue (12) rotatif sur un axe ainsi qu'une pluralité de corps de rotation (14), qui se trouvent sur la circonférence extérieure du corps de roue (12) et qui forment la surface de roulement de la roue (10), **caractérisée en ce que** le corps de roue (12) comporte au moins une cavité, qui comporte au moins un orifice d'entrée et au moins un orifice de sortie pour un liquide de rinçage, **en ce que** l'au moins un orifice de sortie pour le liquide de rinçage est disposé dans la zone des corps de rotation (14) et **en ce que** la roue est conçue pour permettre l'introduction d'un liquide de rinçage par l'orifice d'entrée dans l'au moins une cavité du corps de roue et le baignage des corps de rotation par le liquide de rinçage après la sortie du liquide de rinçage par l'orifice de sortie.

10. Roue multidirectionnelle selon la revendication 9, **caractérisée en ce qu'**une pluralité de cavités, en particulier une pluralité de conduits, est prévue, qui comportent l'au moins un orifice de sortie pour le liquide de rinçage dans la zone des corps de rotation (14).

11. Roue multidirectionnelle selon la revendication 9 ou 10, **caractérisée en ce qu'**exactement un orifice de sortie est prévu par corps de rotation (14) ou **en ce que** plusieurs orifices de sortie sont prévus par corps de rotation (14).

12. Roue multidirectionnelle selon l'une des revendications 9 à 11, **caractérisée en ce que** l'orifice de sortie est réalisé en forme de fente et s'étend de préférence le long du corps de rotation (14), ou **en ce qu'**une pluralité d'orifices de sortie est prévue, qui sont de préférence distribués le long du corps de rotation (14).

13. Dispositif comprenant au moins une source pour un liquide de rinçage, au moins un adaptateur de rinçage (200) en liaison avec la source, qui comporte au moins un orifice d'entrée (201), qui est en liaison avec la source, et au moins un orifice de sortie (203) destiné à l'introduction du liquide de rinçage dans la roue (10), et au moins une roue (10) multidirectionnelle selon l'une des revendications 9 à 12.

14. Dispositif selon la revendication 13, **caractérisé en ce que** l'adaptateur de rinçage (200) comporte au moins une, de préférence une pluralité, de parties tubulaires (204) qui sont en liaison avec la source pour un liquide de rinçage.

15. Dispositif selon la revendication 13 ou 14, **caractérisé en ce que** les orifices de sortie (203) sont espacés les uns des autres dans le sens circonférentiel et/ou dans le sens radial de l'adaptateur de rinçage (200).
